(19) Europäisches Patentamt
European Patent Office
Office européen des brevets

(11) **EP 3 338 775 B1**

(12) **EUROPEAN PATENT SPECIFICATION**

(45) Date of publication and mention
of the grant of the patent:
**25.03.2020 Bulletin 2020/13**

(51) Int Cl.:
***A61K 31/365*** (2006.01)   ***A61P 35/00*** (2006.01)

(21) Application number: **16836466.9**

(86) International application number:
**PCT/CN2016/084686**

(22) Date of filing: **03.06.2016**

(87) International publication number:
**WO 2017/028602 (23.02.2017 Gazette 2017/08)**

(54) **USE OF BUTYLIDENEPHTHALIDE**

VERWENDUNG VON BUTYLIDENPHTHALID

UTILISATION DE BUTYLIDÈNE-PHTHALIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.08.2015 US 201562207226 P**

(43) Date of publication of application:
**27.06.2018 Bulletin 2018/26**

(73) Proprietor: **Everfront Biotech Inc.
New Taipei City (TW)**

(72) Inventors:
• **LIN, Shinn-zong**
**Taichung City**
**Taiwan (TW)**
• **CHIOU, Tzyy-wen**
**Hualien County**
**Taiwan (TW)**
• **HARN, Horng-jyh**
**Taipei City**
**Taiwan (TW)**
• **YEN, Ssu-yin**
**Tainan City**
**Taiwan (TW)**

(74) Representative: **Herrero & Asociados, S.L.
Cedaceros, 1
28014 Madrid (ES)**

(56) References cited:
**EP-A1- 2 952 184        WO-A1-2005/082407
CN-A- 102 579 434        CN-A- 105 030 760
CN-A- 105 267 205        US-A1- 2006 110 469
US-A1- 2007 134 351        US-A1- 2011 183 015**

• **PANG CHENG-YOONG ET AL: "Proteomic-based
identification of multiple pathways underlying
n-butylidenephthalide-induced apoptosis in
LNCaP human prostate cancer cells", FOOD AND
CHEMICAL TOXICOLOGY, PERGAMON, GB, vol.
59, 31 August 2013 (2013-08-31), pages 281-288,
XP009511433, ISSN: 0278-6915, DOI:
10.1016/J.FCT.2013.05.045**

**Description**

**FIELD OF THE INVENTION**

The present invention relates to the use of butylidenephthalide (BP), including the prevention, mitigation and/or inhibition of the growth, migration, invasion and/or metastasis of oral cancer stem cells.

**BACKGROUND OF THE INVENTION**

In medicine, a tumor refers to an abnormal cytopathic effect, which is caused by a reaction of various carcinogenic factors that causes cells in the local body tissues to lose normal growth regulation at the genetic level and results in an abnormal cell proliferation. Those abnormal proliferated cells will aggregate into a mass, and this is called as a "tumor." "Cancer" is the most common type of tumor. The abnormal proliferated "cancer cells" will not only aggregate into a mass, but also spread and metastasize to other tissues or organs. Therefore, cancer is also known as a malignant tumor. The proliferation and metastasis of cancer cells will cause severe abnormalities of physiological function and is very difficult to cure; therefore, cancer is currently the first cause of death worldwide.

Traditional methods for treating cancer include surgery, chemotherapy and radiation therapy, etc. Cancer, however, cannot be cured completely by surgical excision of the mass because the cancer cells not exactly cut off may continuously grow and result in an exacerbation of the patient's condition. Therefore, surgical excision is not used alone in the therapy for cancer, and is usually combined with other therapeutic methods such as chemotherapy and/or radiation therapy. In chemotherapy, chemical drugs (such as an alkylating agent) are used to kill the cancer cells having a high proliferation rate. However, most chemical drugs used in chemotherapies also act on normal cells, resulting in severe side effects (include vomiting, alopecia, fatigue, bleeding, anemia, etc.) to cancer patients. Radiation therapy (e.g., gamma knife radiosurgery) breaks down the DNA of cancer cells on the principle that rapidly dividing cancer cells are more sensitive to radiation than normal cells. However, when high-energy radiation destroys cancer cells, normal cells may also be radiated simultaneously, resulting in side effects such as a loss of leukocytes, fatigue, insomnia, pain, and poor appetite. Furthermore, chemotherapy and radiation therapy are not effective for a part of cancer patients in the late stages.

Research has shown that most cancer cells do not have the ability to induce a tumor and only few cancer cells have tumorigenicity. Those cancer cells with tumorigenicity have characteristics of stem cells (i.e., have the ability of self-renewal and differentiation), and can continuously grow and differentiate into different kinds or types of tumor cells, and thus, are called as "cancer stem cells (CSCs)" or "tumor stem cells". Research has also shown that cancer stem cells have the potential to form a tumor and develop into cancer. In particular, cancer stem cells will develop into other kinds of cancer when metastasizing to other tissues or organs of the body. Furthermore, in the tissues of such as leukemia, breast cancer, brain cancer, ovarian cancer, prostate cancer, colon cancer, rectal cancer, or oral cancer, cancer stem cells are more resistant to chemotherapy or radiation therapy than other cells. Such resistance is highly correlated with the recurrence, invasion, and metastasis of the tumor and survival rate of the patient. Thus, if the growth, migration, invasion and/or metastasis of cancer stem cells can be prevented, mitigated or inhibited effectively, the success rate for treating the tumor and cancer and the survival rate of patient can be improved.

Currently, the therapeutic methods (including surgery, chemotherapy and radiation therapy, etc.) for preventing, mitigating or inhibiting the growth, migration, invasion or metastasis of cancer stem cells in clinic are all inefficient. Therefore, there is a necessity and urgency for developing a method or a drug for preventing, mitigating or inhibiting the growth, migration, invasion or metastasis of cancer stem cells effectively to decrease the incidence rate, recurrent rate, and death rate, as well as to improve the cure rate and reduce side effects.

Inventors of the present invention found that BP is effective in inhibiting the expressions of the growth related factors, stemness factors, epithelial-mesenchymal transition (EMT) factors, and gelatinases of cancer stem cells, especially in inhibiting the expressions of Sox-2, Oct4 and EZH2, and thus, can inhibit the growth, migration, invasion and metastasis of cancer stem cells and can be used to provide a medicament, a food product or a food additive for preventing, mitigating and/or inhibiting the growth, migration, invasion and/or metastasis of cancer stem cells.

**SUMMARY OF THE INVENTION**

The present invention is defined by the appended claims. Thus, the invention refers to:

1. A preparation for use in decreasing the incidence rate and/or recurrent rate of oral cancer by preventing, mitigating and/or inhibiting the growth, migration, invasion and/or metastasis of oral cancer stem cells, comprising an effective amount of butylidenephthalide (BP).
2. The preparation for use as claimed in claim 1, which is for inhibiting the expressions of Sox-2 and Oct4, thereby preventing, mitigating and/or inhibiting the growth, migration and/or invasion of oral cancer stem cell.

3. The preparation for use as claimed in claim 1 or 2, which is a medicament, a food product, or a food additive.

4. The preparation for use as claimed in any one of claims 1 to 3, which is administered at an amount ranging from about 30 mg (as BP)/kg-body weight to about 500 mg (as BP)/kg-body weight per day.

5. The preparation for use as claimed in any one of claims 1 to 3, which is administered at an amount ranging from about 40 mg (as BP)/kg-body weight to about 120 mg (as BP)/kg-body weight per day.

## OTHER ASPECTS OF THE DISCLOSURE

An objective of the present disclosure is to provide a method for preventing, mitigating and/or inhibiting the growth, migration, invasion and/or metastasis of cancer stem cells (CSCs), comprising administering to a subject in need an effective amount of butylidenephthalide (BP).

Specifically, the objective of the present disclosure is to provide a use of butylidenephthalide (BP) in the manufacture of a preparation, wherein the preparation is for preventing, mitigating and/or inhibiting the growth, migration, invasion and/or metastasis of cancer stem cells (CSCs).

Preferably, the preparation is for inhibiting the expressions of Sox-2, Oct4 and EZH2, thereby preventing, mitigating and/or inhibiting the growth, migration, invasion and/or metastasis of cancer stem cell (CSC).

Preferably, the cancer stem cell (CSC) is at least one of oral cancer stem cells, nasopharyngeal cancer stem cells, esophageal cancer stem cells, myeloma stem cells, skin cancer stem cells, melanoma stem cells, thyroid cancer stem cells, lymphoma stem cells, leukemia stem cells, breast cancer stem cells, bladder cancer stem cells, ovarian cancer stem cells, cervical cancer stem cells, prostate cancer stem cells, gastric cancer stem cells, liver cancer stem cells, lung cancer stem cells, colon cancer stem cells, rectal cancer stem cells, pancreatic cancer stem cells, gall bladder cancer stem cells, renal cancer stem cells, glioblastoma stem cells, thymic carcinoma stem cells, rhabdomyosarcoma stem cells, brain tumor stem cells and medulloblastoma stem cells.

Preferably, the cancer stem cell (CSC) is oral cancer stem cell.

Preferably, the cancer stem cell (CSC) is pancreatic cancer stem cell.

Preferably, the cancer stem cell (CSC) is brain tumor stem cell.

Preferably, the preparation is a medicament, a food product, or a food additive.

Preferably, the preparation is a medicament and is administered at an amount ranging from about 30 mg/kg-body weight to about 500 mg/kg-body weight per day.

Preferably, the preparation is administered at an amount ranging from about 40 mg/kg-body weight to about 120 mg/kg-body weight per day.

The detailed technology and preferred embodiments implemented for the present invention and disclosure are described in the following paragraphs accompanying the appended drawings for people skilled in this field to well appreciate the features of the claimed invention.

## BRIEF DESCRIPTION OF THE DRAWINGS

**FIG. 1A and FIG. 1B are** bar diagrams showing the relative viability of $CD133^+$ brain tumor stem cells (hereinafter referred to as "brain CSCs $^{CD133+}$"), wherein FIG. 1A shows the results of brain CSCs $^{CD133+}$ treated with different concentrations of butylidenephthalide (BP), and FIG. 1B shows the results of brain CSCs $^{CD133+}$ treated with different concentrations of bis-chloroethylnitrosourea (BCNU);

**FIG. 2** is a curve diagram showing the relative viability of NHOKs (normal human oral keratinocytes ♦) treated with different concentrations of BP and that of HNC-TIC (head and neck cancer-derived tumor initiating cell)-like oral stem cells (i.e., $ALDH1^+CD44^+$-1 cell line ■ or $ALDH1^+CD44^+$-2 cell line ▲) treated with different concentrations of BP;

**FIG. 3 to FIG. 5** show the results of Western blotting, showing the expressions of the stemness-maintenance markers in brain CSCs $^{CD133+}$ treated with different concentrations of BP, wherein FIG. 3 is a photograph showing the protein expressions of EZH2 and actin, FIG. 4 is a photograph showing the protein expressions of CD 133 and actin in brain CSCs $^{CD133+}$ and FIG. 5 is a photograph showing the protein expressions of Sox-2, Oct4 and β-actin in brain CSCs $^{CD133+}$;

**FIG. 6** is a photograph of the results of Western blotting, showing the protein expressions of Sox-2, CD133, CD44 and β-actin in MiaPaCa-2 cells (i.e., a cell line of pancreatic cancer cells) treated with different concentrations of BP;

**FIG. 7** is a photograph of the results of Western blotting, showing the protein expressions of Oct4, Sox-2 and GAPDH in HNC-TIC-like oral stem cells (i.e., $ALDH1^+CD44^+$-1 cell line or $ALDH1^+CD44^+$-2 cell line) treated with different concentrations of BP;

**FIG. 8** shows the results of flow cytometry analysis, showing the ALDH activity of HNC-TIC-like oral stem cells (i.e.,

ALDH1$^+$CD44$^+$-1 cell line or ALDH1$^+$CD44$^+$-2 cell line) treated with different concentrations of BP in the presence or absence of N,N-diethylaminobenzaldehyde (DEAB);

**FIG. 9A** is a photograph of the results of Western blotting, showing the protein expressions of Axl, Gas 6 and actin in brain CSCs $^{CD133+}$ treated with different concentrations of BP;

**FIG. 9B** is a photograph of the results of reverse transcriptase-polymerase chain reaction (RT-PCR), showing the gene expressions of *Axl* and *GAPDH* in brain CSCs $^{CD133+}$ treated with different concentrations of BP;

**FIG. 10** is a photograph of Western blotting, showing the protein expressions of MMP-2 and actin in brain CSCs $^{CD133+}$ treated with different concentrations of BP;

**FIG. 11A and FIG. 11B** show the results of transwell invasion assay system, showing the invasion ability of HNC-TIC-like oral stem cells (i.e., ALDH1$^+$CD44$^+$-1 cell line or ALDH1$^+$CD44$^+$-2 cell line) treated with different concentrations of BP, wherein FIG. 11A is a photograph showing the staining results of the polycarbonate filter membrane in the lower chamber, and FIG. 11B is a bar diagram showing the quantified results of relative invasion ability;

**FIG. 12** is a photograph of Western blotting, showing the protein expressions of E-cadherin, TGFβ-1, Slug and β-actin in brain CSCs $^{CD133+}$ treated with different concentrations of BP;

**FIG. 13** shows the results of a wound healing assay, showing the migration ability of brain CSCs $^{CD133+}$, wherein the upper row is a photograph showing the results of brain CSCs $^{CD133+}$ treated with different concentrations of BP and the lower row showing the results of brain CSCs $^{CD133+}$ treated with different concentrations of BCNU;

**FIG. 14A** is a photograph taken with the use of a microscope, showing the brain CSCs $^{CD133+}$ having pcDNA 3.0-Axl plasmid (hereinafter referred to as "pcDNA 3.0-Axl brain CSCs $^{CD133+}$") or brain CSCs $^{CD133+}$ having pcDNA3.0-neo plasmid (hereinafter referred to as "pcDNA 3.0-neo brain CSCs $^{CD133+}$");

**FIG. 14B** is a photograph of Western blotting, showing the protein expressions of Axl-1 and β- actin in pcDNA 3.0-Axl brain CSCs $^{CD133+}$ or pcDNA3.0-neo brain CSCs $^{CD133+}$;

**FIG. 14C** shows the results of a wound healing assay, showing the migration ability of pcDNA 3.0-Axl brain CSCs $^{CD133+}$ treated with different concentrations of BP or that of pcDNA3.0-neo brain CSCs $^{CD133+}$ treated with different concentrations of BP;

**FIG. 15A and FIG. 15B** show the results of a soft agar colony formation assay, showing the colony-forming ability of oral cancer stem cells treated with different concentrations of BP, wherein FIG. 15A is a photograph showing the colony formation of ALDH1$^+$CD44$^+$-1 cell line and ALDH1$^+$CD44$^+$-2 cell line, and FIG. 15B is a bar diagram showing the relative colony-forming ability of ALDH1$^+$CD44$^+$-1 cell line and ALDH1$^+$CD44$^+$-2 cell line;

**FIG. 16A to FIG. 16D** show the results of an animal experiment, showing the ability of BP to inhibit the tumor initiating activity of cancer stem cells, including the results of the mice without BP treatment (i.e., "control group," ——●——), the mice treated with 100 mg/kg of BP (i.e., "100 mg/kg group," ——○——), and the mice treated with 200 mg/kg of BP (i.e., "200 mg/kg group, " ——▼——), wherein FIG. 16A shows the average weight (g) of each group of mice over a period of time, FIG. 16B shows the average tumor volume (mm$^3$) of each group of mice over a period of time, FIG. 16C is a photograph showing the GFP intensity (i.e. green fluorescence) in each group of mice, and FIG. 16D is a bar diagram showing the GFP intensity in each group of mice.

## DESCRIPTION OF THE PREFERRED EMBODIMENTS

The following will describe some of the embodiments of the present invention in detail. However, the present invention may be embodied in various embodiments and should not be limited to the embodiments described in the specification. In addition, unless otherwise indicated herein, the expressions "a," "an", "the", or the like recited in the specification of the present invention (especially in the claims) are intended to include the singular and plural forms. The term "effective amount" or "therapeutically effective amount" used in this specification refers to the amount of compound that can at least partially alleviate the condition that is being treated in a suspected subject when administered to the subject in need. The term "subject" used in this specification refers to a mammalian, including human or non-human animals.

Inventors of the present invention found that butylidenephthalide (BP) is effective in inhibiting the viability of cancer stem cells, inhibiting the expressions of growth related factors (e.g., EZH2) of cancer stem cells, inhibiting the expressions of the stemness-maintenance markers (e.g., CD133, Sox-2, Oct4) of cancer stem cells, inhibiting the expressions of genes (e.g., *AXL*) or proteins (Axl, Gas6, MMP-2) related to the proliferation, anti-apoptosis, migration and invasion of cancer stem cells, inhibiting the expressions of proteins related to EMT (epithelial-mesenchymal transition) (e.g., E-cadherin, TGFβ-1 and Slug) of cancer stem cells, and inhibiting the ability of cancer stem cells to migrate to the wound. In some embodiments of the present invention, BP prevented, mitigated and/or inhibited the growth, migration, invasion and/or metastasis of cancer stem cells by inhibiting the expressions of Sox-2, Oct-4 and EZH2.

Therefore, the present invention relates to the use of BP in the manufacture of a preparation, wherein the preparation is for decreasing the incidence rate and/or recurrent rate of oral cancer by preventing, mitigating and/or inhibiting the growth, migration, invasion and/or metastasis of oral cancer stem cells.

The preparation of the present disclosure can be used in any suitable cancer stem cells and the examples of the cancer stem cells include, for example, oral cancer stem cells, nasopharyngeal cancer stem cells, esophageal cancer stem cells, myeloma stem cells, skin cancer stem cells, melanoma stem cells, thyroid cancer stem cells, lymphoma stem cells, leukemia stem cells, breast cancer stem cells, bladder cancer stem cells, ovarian cancer stem cells, cervical cancer stem cells, prostate cancer stem cells, gastric cancer stem cells, liver cancer stem cells, lung cancer stem cells, colon cancer stem cells, rectal cancer stem cells, pancreatic cancer stem cells, gall bladder cancer stem cells, renal cancer stem cells, glioblastoma stem cells, thymic carcinoma stem cells, rhabdomyosarcoma stem cells, brain tumor stem cells and medulloblastoma stem cells. In some embodiments of the present invention, the preparation is used to prevent, mitigate and/or inhibit the growth, migration, invasion and/or metastasis of oral cancer stem cells. In some embodiments of the present disclosure, the preparation is used to prevent, mitigate and/or inhibit the growth, migration, invasion and/or metastasis of pancreatic cancer stem cells and/or brain tumor stem cells.

The preparation in accordance with the present invention can be provided in any suitable form without particular limitations. For example, the preparation can be provided as medicaments, but is not limited thereby. The preparation can also be provided in liquid or solid form as a food product or food additive.

When the preparation according to the present invention is provided as a medicament, the medicament can be prepared in any suitable dosage form depending on the desired administration manner. For example, the medicament can be administered by oral or parenteral (e.g., subcutaneous, intravenous, intramuscular, peritoneal, or nasal) route to a subject in need, but is not limited thereby. Depending on the form and purpose, suitable carriers can be chosen and used to provide the medicament, wherein examples of the suitable carriers include excipients, diluents, auxiliaries, stabilizers, absorbent retarders, disintegrants, hydrotropic agents, emulsifiers, antioxidants, adhesives, binders, tackifiers, dispersants, suspending agents, lubricants, hygroscopic agents, etc.

As a dosage form suitable for oral administration, the medicament provided by the present invention can comprise any pharmaceutically acceptable carrier (e.g., water, saline, dextrose, glycerol, ethanol or its analogs, cellulose, starch, sugar bentonite, or combinations thereof) that will not adversely affect the desired effects of BP. The medicament can be provided in any dosage form suitable for oral administration, such as be provided as a tablet (e.g., dragee), a pill, a capsule, a granule, a pulvis, a fluidextract, a solution, syrup, a suspension, an emulsion, and a tincture, etc.

As for an injection form for subcutaneous, intravenous, intramuscular, or peritoneal administration or a drip form, the medicament provided by the present invention can comprise one or more ingredient(s) such as an isotonic solution, a salt-buffered saline (e.g., phosphate-buffered saline or citrate-buffered saline), a hydrotropic agent, an emulsifier, 5% sugar solution, and other carriers to provide the medicament as an intravenous infusion, an emulsified intravenous infusion, a powder for injection, a suspension for injection, or a powder suspension for injection, etc. Alternatively, the medicament can be prepared as a pre-injection solid. The pre-injection solid can be provided in a form which is soluble in other solutions or suspensions, or in an emulsifiable form. A desired injection is provided by dissolving the pre-injection solid in a solution or a suspension or emulsifying it prior to being administered to a subject in need. In addition, examples of the dosage form for external use which are suitable for nasal or transdermal administration include an emulsion, a cream, gel (e.g., hydrogel), paste (e.g., a dispersion paste, an ointment), a spray, or a solution (e.g., a lotion, a suspension).

Optionally, the medicament provided by the present invention can further comprise a suitable amount of additives, such as a flavoring agent, a toner, or a coloring agent for enhancing the palatability and the visual perception of the medicament, and/or a buffer, a conservative, a preservative, an antibacterial agent, or an antifungal agent for improving the stability and storability of the medicament. In addition, the medicament can optionally further comprise one or more other active ingredients or be used in combination with a medicament comprising one or more other active ingredients, to further enhance the effects of the medicament or to increase the application flexibility and adaptability of the formulation thus provided, as long as the other active ingredient(s) will not adversely affect the desired effects of BP.

Depending on the age, body weight, and health conditions (such as the disease to be treated and its severity) of the subject to be administrated, the medicament provided by the present invention can be applied with various administration frequencies, such as once a day, multiple times a day, or once every few days, etc. For example, when the medicament is applied orally to a subject for preventing, mitigating and/or inhibiting the growth, migration, invasion and/or metastasis of cancer stem cells, the dosage of the medicament is about 30 mg (as BP)/kg-body weight to about 500 mg (as BP)/kg-body weight per day, preferably about 40 mg (as BP)/kg-body weight to about 120 mg (as BP)/kg-body weight per day, and more preferably about 50 mg (as BP)/kg-body weight to about 90 mg (as BP)/kg-body weight per day, wherein the unit "mg/kg-body weight" refers to the dosage required per kg-body weight of the subject. However, for acute patients, the dosage may be optionally increased up to such as several folds or dozen fold, depending on the practical requirements.

Optionally, the medicament of the present invention can be used in combination with surgery, chemotherapy, radiation therapy, antibody therapy, immunotherapy, anti-angiogenesis therapy, phenotype conversion and/or differentiation therapy, to prevent, mitigate and/or inhibit the growth, migration, invasion and/or metastasis of cancer stem cells.

When the preparation according to the present invention is provided as a food additive, the food additive can be in a form that could be conveniently added during the food manufacturing processes, such as in the form of powder, liquid, suspension or granule. When the preparation according to the present invention is provided as a food product, the food product can be such as milk products, processed meat, breads, pasta products, biscuits, troches, juices, teas, sport drinks, nutritious drinks, and the likes, and can be heath food (e.g., nutritional supplements after surgery), but is not limited thereby.

Depending on the age, body weight and healthy conditions of the subject to be administrated, the health food provided by the present invention can be taken in various frequencies, such as once a day, several times a day or once every few days, etc. The amount of BP in the health food provided by the present invention can be adjusted, preferably to the amount that should be taken daily, depending on the specific population. For example, if the recommended daily dosage for a subject is about 50 mg and each serving of the health food contains 25 mg of BP, the subject can take about two servings of the health food per day.

The recommended daily dosages, use standards and use conditions for a specific population (e.g., pregnant woman and diabetic patients), or recommendations for a use in combination with another food or medicament can be indicated on the outer package of the health food of the present invention, and thus, is favorable for user to take the health food by him- or herself safely and securely without the instructions of a doctor, pharmacist, or related executive.

The present disclosure also provides a method for preventing, mitigating and/or inhibiting the growth, migration, invasion and/or metastasis of cancer stem cells (CSCs), comprising administering to a subject in need an effective amount of butylidenephthalide (BP). In this method, the applied form and suitable dosage of BP and suitable cancer stem cells are all in line with the above description about the preparation of the present invention.

The present invention will be further illustrated in detail with specific examples as follows. However, the following examples are provided only for illustrating the present invention, and the scope of the present invention is not limited thereby. The scope of the present invention is shown in the claims.

## EXAMPLES

### Example 1: Effect of BP on inhibiting the growth of cancer stem cells

#### (1-1)

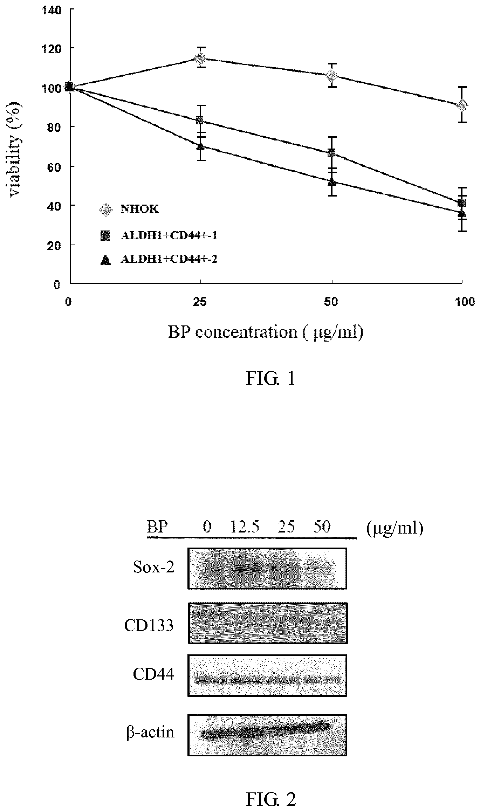

To investigate the effect of BP on inhibiting the growth of cancer stem cells, brain CSCs [CD133+] or malignant glioma DBTRG cells were treated with different concentrations (0, 25, 50, 62.5, 75 and 100 μg/ml) of BP, different concentrations (0, 25, 125, 250, 500 and 1000 μM) of bis-chloroethylnitrosourea (BCNU, a currently used chemotherapy drug for treating malignant glioma in clinic), or different concentrations (0, 100, 200, 400, 800 and 1600 μM) of temozolomide (TMZ, a currently used chemotherapy drug for treating malignant glioma in clinic) for 24 or 48 hours. Thereafter, a cell viability assay, MTT assay, was utilized to analyze the viabilities of brain CSCs [CD133+] and malignant glioma DBTRG cells. The viability of each experimental group was calculated based on the result of the control group, i.e., the group without being treated with BP, BCNU or TMZ. The results are shown in Figure 1A and Figure 1B for 24-hour group and 48-hour group (i.e., the treatment for 24 hours and 48 hours) and in Table 1 for 24-hour group.

Table 1

|  | $IC_{50}$ to brain CSCs [CD133+] | $IC_{50}$ to malignant glioma DBTRG cells |
|---|---|---|
| BP | 406 μM (i.e., 76.5 μg/ml) | 400 μM (i.e., 75.3 μg/ml) |
| BCNU | 377.6 μM (i.e., 80.8 (μg/ml) | 352.4 μM (i.e., 75.4 μg/ml) |
| TMZ | > 1,600 μM (i.e., > 310.6 μg/ml) | 1,658.6 μM (i.e., 322.0 μg/ml) |

As shown in Figure 1A and Figure 1B, in both the 24-hour group and 48-hour group, the viability of brain CSCs [CD133+] significantly decreased along with the increment in the concentration of BP or BCNU. On the other hand, as shown in Table 1, the half maximal inhibitory concentration ($IC_{50}$) of BP, BCNU, or TMZ to brain CSCs [CD133+] in the 24-hour group was 406 μM (i.e., 76.5 μg/ml), 377.6 μM (i.e., 80.8 μg/ml), and greater than 1,600 μM (i.e., > 310.6 μg/ml), respectively. These results indicate that BP is effective in inhibiting the growth of cancer stem cells and its effect is superior to that of currently used chemotherapy drugs.

**(1-2)**

NHOKs (normal human oral keratinocytes) and HNC-TIC (head and neck cancer-derived tumor initiating cell)-like oral stem cells (i.e., ALDH1+CD44+-1 cell line or ALDH1+CD44+-2 cell line) were treated with different concentrations (0, 25, 50 and 100 $\mu$g/ml) of BP for 24 hours, and then, an MTT assay was utilized to analyzed the viabilities of NHOKs and HNC-TIC-like oral stem cells (i.e., ALDH1+CD44+-1 cell line and ALDH1+CD44+-2 cell line). The results are shown in Figure 2.

As shown in Figure 2, the viability of NHOKs did not decrease along with the increment in the concentration of BP, while that of HNC-TIC-like oral stem cells (i.e., ALDH1+CD44+-1 cell line and ALDH1+CD44+-2 cell line) decreased along with the increment in the concentration of BP. These results indicate again that BP is effective in inhibiting the growth of cancer stem cells and has no cytotoxicity to normal cells. Therefore, BP will not cause cytotoxicity-induced side effects and thus can be used to provide a quality drug for inhibiting the growth of cancer stem cells.

**Example 2: Effect of BP on inhibiting the expressions of growth related factors of cancer stem cells**

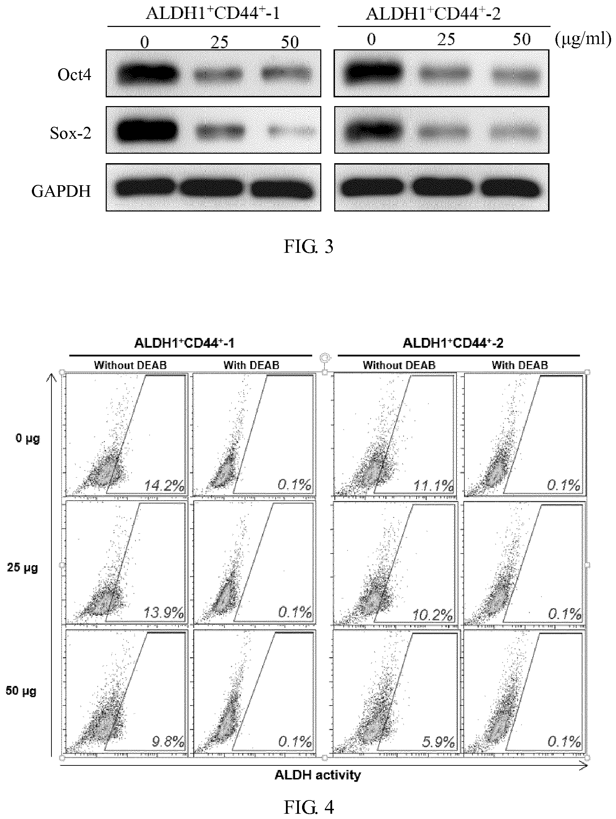

It has been known that *EZH2 (enhancer of zeste homolog 2)* gene is a critical growth related factor of cancer stem cells, and it will affect the growth and growth characteristics of cancer stem cells. Brain CSCs CD133+ was treated with different concentrations (0, 12.5, 25, 50, 62.5 and 75 $\mu$g/ml) of BP, and then, proteins were extracted from the cells to conduct Western blotting to determine the expression of EZH2 protein in the BP-treated brain CSCs CD133+. The results are shown in Figure 3.

As shown in Figure 3, the expression of EZH2 protein in brain CSCs CD133+ significantly decreased along with the increment in the concentration of BP. These results indicate again that BP is effective in inhibiting the growth of cancer stem cells.

**Example 3: Effect of BP on inhibiting the sternness of cancer cells and cancer stem cells**

**(3-1)**

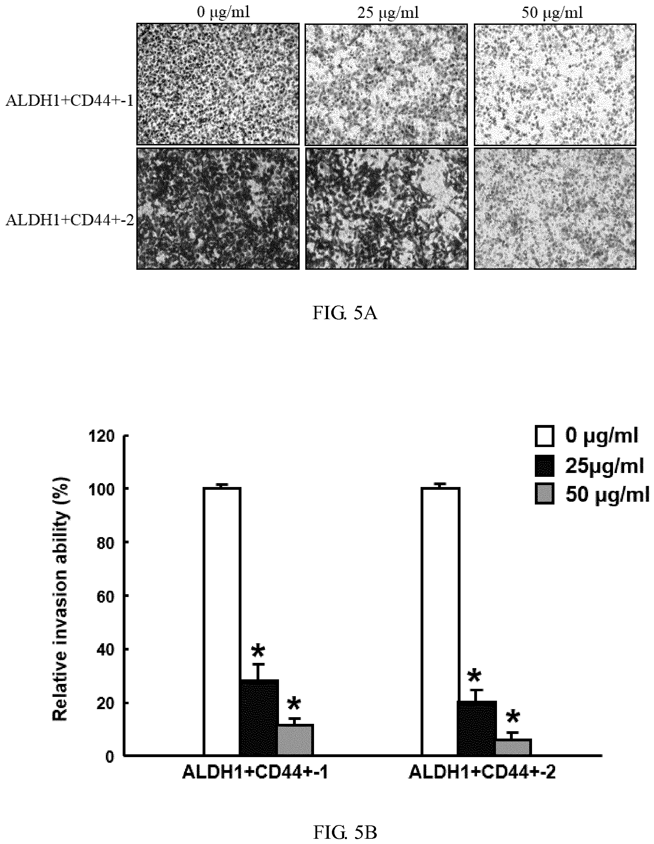

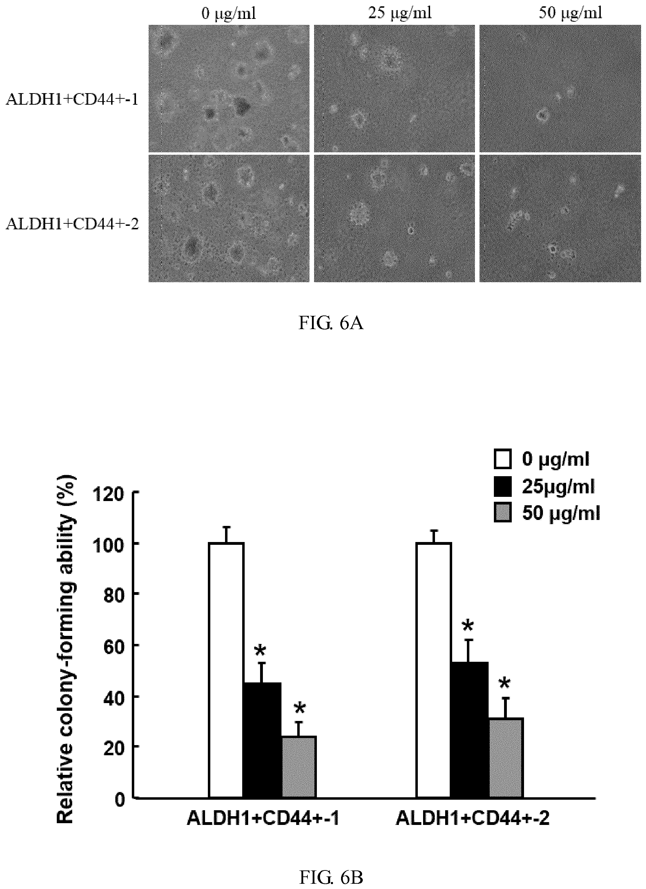

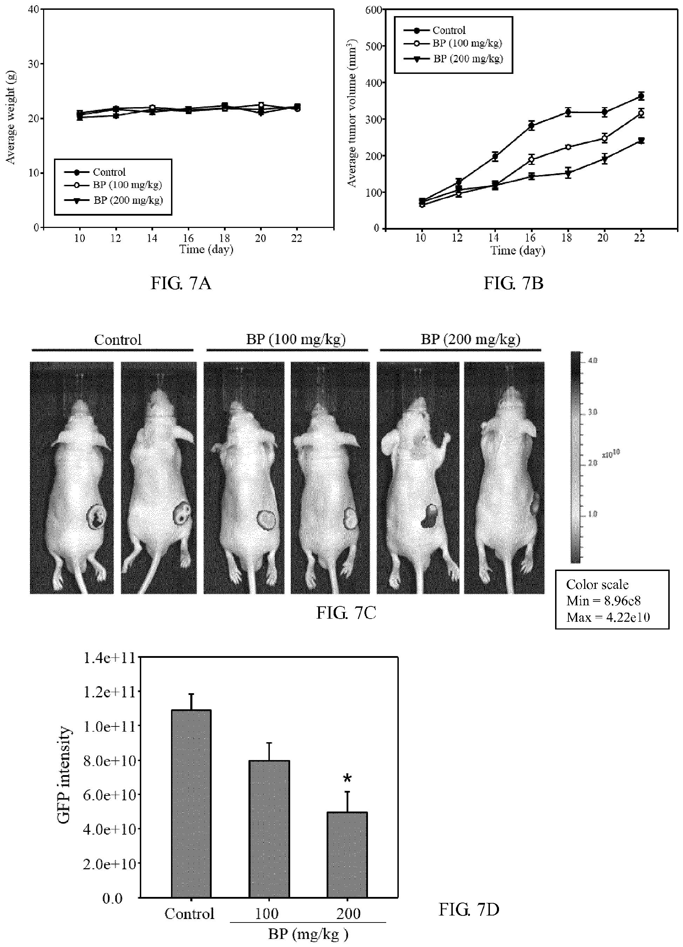

It has been known that CD44, CD133, Sox-2 (sex-determining region Y protein (SRY)-related high-mobility group box 2) and Oct4 (octamerbinding transcription factor 4) proteins all are stemness-maintenance markers of cancer stem cells and participate in the regulation of the self-renewal and differentiation of cancer stem cells. Brain CSCs CD133+ was treated with different concentrations (0, 12.5, 25, 50, 62.5 and 75 $\mu$g/ml) of BP for 24 hours, and then, proteins were extracted from the cells to conduct Western blotting to determine the expressions of CD133, Sox-2 and Oct4 proteins in the BP-treated brain CSCs CD133+. The results are shown in Figure 4 and Figure 5. In addition, MiaPaCa-2 cell line (i.e., a cell line of pancreatic cancer cells) was treated with different concentrations (0, 12.5, 25, and 50 $\mu$g/ml) of BP for 24 hours, and then, proteins were extracted from the cells to conduct Western blotting to determine the expressions of Sox-2, CD133 and CD44 proteins in the BP-treated MiaPaCa-2 cells. The results are shown in Figure 6. Yet another, HNC-TIC-like oral stem cells (i.e., ALDH1+CD44+-1 cell line or ALDH1+CD44+-2 cell line) were treated with different concentrations (0, 12.5, 25, and 50 $\mu$g/ml) of BP for 24 hours, and then, proteins were extracted from the cells to conduct Western blotting to determine the expressions of Sox-2 and Oct4 proteins in the BP-treated HNC-TIC-like oral stem cells (i.e., ALDH1+CD44+-1 cell line and ALDH1+CD44+-2 cell line). The results are shown in Figure 7.

As shown in Figure 4 and Figure 5, the expressions of CD133, Sox-2 and Oct4 proteins in brain CSCs CD133+ significantly decreased along with the increment in the concentration of BP. As shown in Figure 6, the expressions of Sox-2, CD133 and CD44 proteins in MiaPaCa-2 cells significantly decreased along with the increment in the concentration of BP. As shown in Figure 7, the expressions of Sox-2 and Oct4 proteins in the HNC-TIC-like oral stem cells (i.e., ALDH1+CD44+-1 cell line and ALDH1+CD44+-2 cell line) significantly decreased along with the increment in the concentration of BP. These results indicate that BP is effective in inhibiting the stemness of cancer cells and cancer stem cells.

**(3-2)**

ALDH protein is also an important stemness-maintenance marker of cancer stem cells and participates in the regulation of the self-renewal and differentiation of cancer stem cells. HNC-TIC-like oral stem cells (i.e., ALDH1+CD44+-1 cell line or ALDH1+CD44+-2 cell line) were treated with different concentrations (0, 25 and 50 $\mu$g/ml) of BP for 24 hours, and then, $1 \times 10^5$ cells obtained from each group were separately suspended in 50 $\mu$l of a stem cell detection kit (i.e., ALDEFLUOR array kit, purchased from STEMCELL Technologies Inc., Vancouver, Canada) buffer and the cell suspension of each group was separately added with ALDEFLUOR to a final concentration of 1 $\mu$M. Thereafter, a sample of each group was stained by 7-ADD and then analyzed by flow cytometry analysis to determine the ALDH activity of HNC-TIC-like oral stem cells (i.e., ALDH1+CD44+-1 cell line and ALDH1+CD44+-2 cell line). The results are shown in Figure 8.

The above experimental procedure was repeated, but the cell suspension of each group was further added with N,N-diethylaminobenzaldehyde (DEAB, i.e., an inhibitor of ALDH) to a final concentration of 150 $\mu$M of DEAB separately. The results are also shown in Figure 8.

As shown in Figure 8, in the DEAB-added group, no matter there was a treatment of BP or not, the live cells with ALDH activity were only 0.1 % of total live cells. On the other hand, in the group without DEAB addition, the number of live cells with ALDH activity increased along with the increment in the concentration of BP. These results indicate that BP is effective in inhibiting the ALDH activity of cancer stem cells, and prove again that BP is effective in inhibiting stemness of cancer stem cells.

**Example 4: Effect of BP on inhibiting the proliferation, anti-apoptosis, migration and invasion of cancer stem cells**

**(4-1)**

It has been known that the expression of Axl (i.e., a receptor tyrosine kinase) is related to the ability of cancer stem cells in proliferation, anti-apoptosis, migration and invasion. Brain CSCs [CD133+] was treated with different concentrations (0, 12.5, 25, 50, 62.5 and 75 $\mu$g/ml) of BP for 24 hours, and then, proteins and total RNA were extracted from the cells to conduct Western blotting and reverse transcriptase-polymerase chain reaction (RT-PCR) to determine the expressions of Axl and Gas6 (i.e., a ligand of Axl) proteins and the expression of *Axl* gene in the BP-treated brain CSCs [CD133+]. The results are shown in Figure 9A and Figure 9B.

As shown in Figure 9A and Figure 9B, the expressions of Axl and Gas6 proteins and the expression of *Axl* gene all significantly decreased along with the increment in the concentration of BP. These results indicate that BP is effective in inhibiting the proliferation, anti-apoptosis, migration and invasion of cancer stem cells.

**(4-2)**

It has been known that at the start of the invasion of cancer stem cells, the extracellular matrix (ECM) is degraded by matrix metalloproteinases (MMPs). Wherein, the expressions of gelatinases, such as gelatinase-A (i.e., MMP-2) and gelatinase-B (i.e., MMP-9), are highly related to the histopathology of tumor metastasis. Brain CSCs [CD133+] was treated with different concentrations (0, 12.5, 25, 50, 62.5 and 75 $\mu$g/ml) of BP for 24 hours, and then, proteins were extracted from the cells to conduct Western blotting to determine the expression of MMP-2 protein in the BP-treated brain CSCs [CD133+]. The results are shown in Figure 10.

As shown in Figure 10, the expression of MMP-2 protein significantly decreased along with the increment in the concentration of BP. These results indicate again that BP is effective in inhibiting the migration and invasion of cancer stem cells.

**(4-3)**

In this research, the effect of BP on inhibiting the migration and invasion of cancer stem cells was further investigated by a transwell invasion assay system (i.e., Transwell[○,R] system, purchased from Corning, UK) with a polycarbonate filter membrane of 8-$\mu$m pore size (purchased from Coming, UK). First, the polycarbonate filter membrane was coated with Matrigel™ (purchased from BD Pharmingen, USA), and then, the coated membrane was placed in the lower chamber of the transwell invasion assay system. Then, the lower chamber was filled with 10% serum-containing medium. On the other hand, HNC-TIC-like oral stem cells (i.e., ALDH1$^+$CD44$^+$-1 cell line or ALDH1$^+$CD44$^+$-2 cell line) were treated with different concentrations (0, 25 and 50 $\mu$g/ml) of BP for 24 hours, and then, the treated cells of each group was cultured in the upper chamber which was filled with serum-free medium, at a cell density of $1\times10^5$ cells/100 $\mu$l for 24 hours. Lastly, the medium of the lower chamber was removed, and the polycarbonate filter membrane in the lower chamber was taken out, fixed with 4 % formalin and stained with crystal violet. The invasion cancer cells of each group were counted and photographed over five visual fields of the membrane with the use of a 100-fold magnification under a microscope. The results are shown in Figure 11A. The relative invasion ability of cells of each experimental group was calculated based on the result of the group treated with 0 $\mu$g/ml of BP. The results are shown in Figure 11B.

As shown in Figure 11A, the number of cells on the polycarbonate filter membrane significantly reduced along with the increment in the concentration of BP. As shown in Figure 11B, the relative invasion ability of cancer stem cells also decreased along with the increment in the concentration of BP. These results indicate again that BP is effective in inhibiting the migration and invasion of cancer stem cells.

**Example 5: Effect of BP on inhibiting the metastasis of cancer stem cells**

**(5-1)**

In has been known that at the start of the epithelial-mesenchymal transition (EMT) of cancer stem cells, the expression of E-cadherin would reduce and the aggregation force between cancer stem cells would decrease, so that the cancer stem cells would gain great invasion and migration capacity and depart from the primary tumor, and thus, could metastasize. Brain CSCs CD133+ was treated with different concentrations (0, 12.5, 25, 50, 62.5 and 75 μg/ml) of BP for 24 hours, and then, proteins were extracted from the cells to conduct Western blotting to determine the expressions of E-cadherin, TGFβ-1 (with an ability to induce EMT) and Slug (i.e., a member of Snail family transcriptional repressors with a property of promoting the metastasis of cancer cells) proteins in the BP-treated brain CSCs CD133+. The results are shown in Figure 12.

As shown in Figure 12, the expressions of TGFβ-1 and Slug proteins significantly decreased along with the increment in the concentration of BP, while the expression of E-cadherin protein significantly increased along with the increment in the concentration of BP. These results indicate that BP up-regulates the expression of E-cadherin protein by inhibiting the expressions of TGFβ-1 and Slug proteins, and thus, can be used to inhibit the onset of EMT of cancer stem cells and inhibit the metastasis of cancer stem cells.

**(5-2)**

In this research, a wound healing assay was used to further investigate the effect of BP on inhibiting the metastasis of cancer stem cells. First, brain CSCs CD133+ was treated with different concentrations (0, 12.5. 25, 50, 62.5 and 75 μg/ml) of BP or different concentrations (0, 25, 50, 100, 200 and 400 μM) of BCNU for 24 hours. At the same time, the phenomenon of brain CSCs CD133+ migrates to the wound was observed and photographed at 0 and 24 hours during the BP treatment or BCNU treatment, respectively. The results are shown in Figure 13.

As shown in Figure 13, the number of BCNU-treated brain CSCs CD133+ migrated to the wound did not decrease along with the increment in the concentration of BCNU. As compared to BCNU-treated brain CSCs CD133+, the number of BP-treated brain CSCs CD133+ migrated to the wound significantly decreased along with the increment in the concentration of BP. These results indicate that BP is capable of inhibiting the metastasis of cancer stem cells.

**(5-3)**

In this research, to further investigate whether the capability of BP in inhibiting the metastasis of cancer stem cells is related to Axl, brain CSCs CD133+ was transfected with pcDNA3.0-Axl plasmid and pcDNA3.0-neo plasmid respectively by using Lipofectmine 2000 transfection reagent. Then, the transfected brain CSCs CD133+ and un-transfected brain CSCs CD133+ were selected by 200 to 600 μg/ml G418 at the same time to obtain brain CSCs CD133+ having pcDNA3.0-Axl plasmid (hereinafter referred to as "pcDNA 3.0-Axl brain CSCs CD133+") or brain CSCs CD133+ having pcDNA3.0-neo plasmid (hereinafter referred to as "pcDNA3.0-neo brain CSCs CD133+"). Both pcDNA 3.0-Axl brain CSCs CD133+ and pcDNA3.0-neo brain CSCs CD133+ were observed and photographed with the use of a microscope. The results are shown in Figure 14A. Then, proteins were extracted from both cells to conduct Western blotting to determine the expressions of Axl protein and β-actin in the BP-treated brain CSCs CD133+. The results are shown in Figure 14B. Yet another, pcDNA 3.0-Axl brain CSCs CD133+ and pcDNA3.0-neo brain CSCs CD133+ were treated with different concentrations (0, 25, 50, 62.5, 75 and 100 μg/ml) of BP for 24 hours. At the same time, the phenomenon of pcDNA 3.0-Axl brain CSCs CD133+ and pcDNA3.0-neo brain CSCs CD133+ migrate to the wound was observed and photographed at 0 and 24 hours during the BP treatment. The results are shown in Figure 14C.

As shown in Figure 14C, the number of pcDNA3.0-neo brain CSCs CD133+ (i.e., brain CSCs CD133+ without Axl overexpression) migrated to the wound significantly decreased along with the increment in the concentration of BP. While, as compared to pcDNA3.0-neo brain CSCs CD133+, the phenomenon that BP suppressing the number of pcDNA 3.0-Axl brain CSCs CD133+ (i.e., brain CSCs CD133+ with Axl overexpression) migrated to the wound was not quite significant. These results and the results of Example 4 indicate that BP can inhibit the metastasis of cancer stem cells through inhibiting the expression of Axl.

**Example 6: Effect of BP on inhibiting the tumor initiating activity of cancer stem cells**

**(6-1)**

In this research, a soft agar colony formation assay was used to investigate the effect of BP on inhibiting the tumor initiating activity of cancer stem cells. First, HNC-TIC-like oral stem cells (i.e., ALDH1+CD44+-1 cell line or ALDH1⁺CD44⁺-2 cell line) were treated with different concentrations (0, 25 and 50 $\mu$g/ml) of BP for 24 hours, and then, the treated cells of each group was separately seeded in the top agar [containing DMEM (Dulbecco's Modified Eagle's Medium medium), 10 % (v/v) Fetal Calf Serum (FCS) and 0.3 % (w/v) agar] (purchased from Sigma-Aldrich) at an initial cell number of $2 \times 10^4$. On the other hand, a six-well culture dish was coated with the bottom agar [containing DMEM, 10 % (v/v) FCS and 0.6 % (w/v) agar] (purchased from Sigma-Aldrich), 2 ml for each well. After the bottom agar was solidified, each group of the top agar (containing oral cancer stem cells) was respectively added onto the bottom agar in different wells of the dish, 2 ml for each well. Thereafter, the culture dish was placed in an 37°C incubator for 4 weeks. Lastly, the bottom agar was stained with 0.005 % crystal violet, the number of colonies (with a diameter $\geq$100 $\mu$m) was counted over five fields per well with the use of a microscope, and those fields were photographed. The results are shown in Figure 15A. The relative colony-forming ability of cells of each experimental group was calculated based on the result of the group treated with 0 $\mu$g/ml of BP. The results are shown in Figure 15B.

As shown in Figure 15A, the number of colonies in the bottom agar significantly decreased along with the increment in the concentration of BP. As shown in Figure 15B, the relative colony-forming ability of cancer stem cells also decreased along with the increment in the concentration of BP. These results indicate that BP is effective in inhibiting the tumor initiating activity of cancer stem cells.

**(6-2)**

In this research, the effect of BP on inhibiting the tumor initiating activity of cancer stem cells was further investigated by animal experiments. First, 5-6 weeks old BALB/c nu/nu mice (weight 18-22 g) were bred under the same condition for 4 weeks. Then, HNC-TIC-like oral stem cells stably transfected with GFP-tagged constructs were injected ($1 \times 10^4$ cells/0.1 mL/mouse) subcutaneously into the axilla of mice. After tumors reached 100 mm³, mice were grouped into one control group and two experimental groups (three groups in total, n=3 per group). Then, one experimental group was injected daily with 100 mg/kg of BP for 6 days and another experimental group was injected daily with 200 mg/kg of BP for 6 days. The control group was injected daily with a vehicle (without BP) for 6 days. Ten days after the injection of BP or vehicle, the length and width were measured every 2 days (22 days in total), and then, the average tumor volume was calculated according to the following Formula 1. The results are shown in Figure 16A and Figure 16B. At the same time, the GFP signal (i.e., green fluorescence) of each group was detected and photographed by the IVIS Imaging system. The results are shown in Figure 16C. The GFP intensity of each group was analyzed by Image-Pro Plus software. The results are shown in Figure 16D.

$$\text{Formula 1: } [\text{length} \times \text{width}^2]/2 \text{ (unit: mm}^3)$$

As shown in Figure 16A to Figure 16D, under the condition that the body weight of mice didn't change significantly, as compared to the control group without BP injection, the GFP intensity of the two experimental groups significantly decreased, and the tumor volume of the two experimental groups decreased over a period of time after the BP injection. These results indicate again that BP is effective in inhibiting the tumor initiating activity of cancer stem cells.

As shown by the above experimental results, BP is effective in inhibiting the viabilities of cancer stem cells, inhibiting the expressions of growth related factors of cancer stem cells, inhibiting the expressions of the stemness-maintenance markers of cancer stem cells, inhibiting the expressions of the genes/proteins related to the proliferation, anti-apoptosis, migration and invasion of cancer stem cells, inhibiting the expressions of the genes/proteins related to the metastasis of cancer stem cells, and inhibiting the abilities of cancer stem cells to migrate to the wound. In particular, BP is effective in inhibiting the expressions of Sox-2, Oct4 and EZH2, and thus, can be used to prevent, mitigate and/or inhibit the growth, migration, invasion and/or metastasis of cancer stem cells.

**Claims**

1. A preparation for use in decreasing the incidence rate and/or recurrent rate of oral cancer by preventing, mitigating and/or inhibiting the growth, migration, invasion and/or metastasis of oral cancer stem cells, comprising an effective amount of butylidenephthalide (BP).

2. The preparation for use as claimed in claim 1, which is for inhibiting the expressions of Sox-2 and Oct4, thereby preventing, mitigating and/or inhibiting the growth, migration and/or invasion of oral cancer stem cell.

3. The preparation for use as claimed in claim 1 or 2, which is a medicament, a food product, or a food additive.

**4.** The preparation for use as claimed in any one of claims 1 to 3, which is administered at an amount ranging from about 30 mg (as BP)/kg-body weight to about 500 mg (as BP)/kg-body weight per day.

**5.** The preparation for use as claimed in any one of claims 1 to 3, which is administered at an amount ranging from about 40 mg (as BP)/kg-body weight to about 120 mg (as BP)/kg-body weight per day.

## Patentansprüche

**1.** Zubereitung zur Verwendung bei der Verringerung der Inzidenzrate und/oder Rezidivrate von Mundkrebs durch Verhindern, Abschwächen und/oder Hemmen des Wachstums, der Migration, Invasion und/oder Metastase von Mundkrebs-Stammzellen, die eine wirksame Menge von Butylidenphthalid (BP) umfasst.

**2.** Zubereitung zur Verwendung nach Anspruch 1 zur Hemmung der Expressionen von Sox-2 und Oct4, wodurch das Wachstum, die Migration und/oder Invasion von Mundkrebs-Stammzellen verhindert, abgeschwächt und/oder gehemmt wird.

**3.** Zubereitung zur Verwendung nach Anspruch 1 oder 2, die ein Arzneimittel, ein Lebensmittelprodukt oder ein Lebensmittelzusatzstoff ist.

**4.** Zubereitung zur Verwendung nach einem der Ansprüche 1 bis 3, die in einer Menge in einem Bereich von etwa 30 mg (als BP)/kg Körpergewicht bis etwa 500 mg (als BP)/kg Körpergewicht pro Tag verabreicht wird.

**5.** Zubereitung nach einem der Ansprüche 1 bis 3, die in einer Menge in einem Bereich von etwa 40 mg (als BP)/kg Körpergewicht bis etwa 120 mg (als BP)/kg Körpergewicht pro Tag verabreicht wird.

## Revendications

**1.** Préparation pour son utilisation dans la réduction du taux d'incidence et/ou du taux de récurrence du cancer de la cavité buccale par prévention, mitigation et/ou inhibition de la croissance, de la migration, de l'invasion et/ou de la métastase de cellules souches de cancer de la cavité buccale, comprenant une quantité efficace de butylidène phtalide (BP).

**2.** Préparation pour son utilisation selon la revendication 1, qui est destinée à l'inhibition des expressions de Sox-2 et Oct4, permettant ainsi la prévention, la mitigation et/ou l'inhibition de la croissance, de la migration et/ou de l'invasion de cellules souches de cancer de la cavité buccale.

**3.** Préparation pour son utilisation selon la revendication 1 ou 2, qui est un médicament, un produit alimentaire ou un additif alimentaire.

**4.** Préparation pour son utilisation selon l'une quelconque des revendications 1 à 3, qui est administrée en une quantité dans la plage d'environ 30 mg (sous la forme de BP)/kg de poids corporel à environ 500 mg (sous la forme de BP)/kg de poids corporel par jour.

**5.** Préparation pour son utilisation selon l'une quelconque des revendications 1 à 3, qui est administrée en une quantité dans la plage d'environ 40 mg (sous la forme de BP)/kg de poids corporel à environ 120 mg (sous la forme de BP)/kg de poids corporel par jour.

FIG. 1

FIG. 2

FIG. 3

FIG. 4

13

FIG. 5A

FIG. 5B

FIG. 6A

FIG. 6B

FIG. 7A

FIG. 7B

FIG. 7C

Color scale
Min = 8.96e8
Max = 4.22e10

FIG. 7D